# EUROPÄISCHE PATENTANMELDUNG

(11) **EP 3 025 849 A1**
(43) Veröffentlichungstag der Anmeldung: **01.06.2016**
(21) Anmeldenummer: 15000948.8
(22) Anmeldetag: 01.04.2015
(51) Int. Cl.: B29C 65/04, H05B 6/50, H05B 6/62, A61M 39/08

(54) **KUNSTSTOFFSCHLAUCHSCHWEISSGERÄT**

(30) Priorität: 25.11.2014 DE 102014017425
(71) Anmelder: Valtronic Technologies (Holding) SA, 1343 Les Charbonnières (CH)
(72) Erfinder: Lepple-Wienhues, Albrecht Dr., 25300 Pontarlier (FR)
(74) Vertreter: Patentanwälte Ruff, Wilhelm, Beier, Dauster & Partner mbB

(57) **Zusammenfassung**

Die Erfindung bezieht sich auf ein Kunststoffschlauchschweißgerät mit einem Zangenteil, das zwei relativ zueinander bewegliche Zangenbacken (12a, 12b) zum Einlegen und Zusammendrücken eines Blutschlauchs (5) aufweist, wobei die Zangenbacken Hochfrequenz(HF)-Elektroden (12a, 12b) beinhalten, und mit einem elektrischen HF-Energieversorgungsschaltkreis, der einen HF-Generator und die HF-Elektroden der Zangenbacken umfasst.

Das erfindungsgemäße Kunststoffschlauchschweißgerät umfasst eine Impedanzsteuereinrichtung zur laufenden Messung und zur Konstanthaltung der Impedanz des HF-Energieversorgungsschaltkreises im Verlauf des jeweiligen Schweißvorgangs. Dazu weist der HF-Generator einen impedanzveränderlichen HF-Schwingkreis mit einer Kondensatoreinheit (13) und einer Spuleneinheit (14) auf, wobei die Kapazität der Kondensatoreinheit (13) und/oder die Induktivität der Spuleneinheit und/oder der ohmsche Widerstand des HF-Schwingkreises variabel einstellbar ist.

Ein derartiges Kunststoffschlauchschweißgerät kann beispielsweise bei einem Verschweißen von Blutschläuchen bei Blutspendebeuteln Verwendung finden.

## Beschreibung

Die Erfindung bezieht sich auf ein Kunststoffschlauchschweißgerät mit einem Zangenteil, das zwei relativ zueinander bewegliche Zangenbacken zum Einlegen und Zusammendrücken eines Kunststoffschlauchs aufweist, wobei die Zangenbacken Hochfrequenz(HF)-Elektroden beinhalten, und mit einem elektrischen HF-Energieversorgungsschaltkreis, der einen HF-Generator und die HF-Elektroden der Zangenbacken umfasst.

Kunststoffschlauchschweißgeräte dieser Art sind verschiedentlich in Gebrauch, beispielsweise als Blutschlauchschweißgeräte, die dazu dienen, zu Blutbeuteln führende Blutschläuche beim Blutspenden nach abgeschlossenem Blutspendevorgang mittels Hochfrequenzenergie zu verschweißen und auf diese Weise luft- und keimdicht abzuschließen. Die Geräte können grundsätzlich als Standgeräte ausgeführt sein, bevorzugt ist jedoch zum Beispiel bei der erwähnten Blutspendeanwendung eine Ausführung als Handgerät mit einem handgehaltenen Gerätekörper, da das Verschweißen aus Hygienegründen am Spender vor Entfernen der Venenkanüle vorgenommen wird. Die herkömmlichen Geräte besitzen typischerweise einen kabelgebundenen Anschluss an eine elektrische Energieversorgungsquelle, wie einen stationären oder schultergetragenen Batteriepack. Handgeräte dieser Art sind beispielsweise das unter dem Markennamen CompoSeal auf dem Markt befindliche Gerät Fresenius CompoSeal Mobilia II und das in der Patentschrift US 5.750.951 offenbarte Gerät.

Es ist bekannt, dass sich bei diesen Geräten während eines jeweiligen Schweißvorgangs die Impedanz des HF-Energieversorgungsschaltkreises ändert, wenn sich der Abstand der HF-Elektroden der Zangenbacken und der (Quetsch-)Zustand des Blutschlauchs zwischen den Zangenbacken ändert, was die Effektivität des Schweißvorgangs und die Qualität der Verschweißungsstelle beeinträchtigen kann. In der besagten US 5.750.971 wird als Abhilfe eine spezielle Zeitsteuerung des Schweißvorgangs unter Verwendung eines Endpunktdetektors und eines vorgebbaren Pfades innerhalb eines Smith-Diagramms vorgeschlagen, wodurch die Dauer des Schweißvorgangs variabel in Abhängigkeit von der sich während des Schweißvorgangs ändernden Impedanz eingestellt wird.

In der Patentschrift US 7.586.071 B2 ist eine stationäre HF-Schweißanlage zum Verschweißen insbesondere von Hüllen und Einbänden aus Kunststoffmaterial wie PVC, PU, PET, PETG oder Polyolefin offenbart. Bei dieser Schweißanlage bewegt sich eine obere Druckplatte gegenüber einer unteren Druckplatte, die als HF-Elektroden fungieren und gleichzeitig Prägehälften für eine zusätzliche Prägefunktion des Schweißvorgangs bilden können. Hierbei kann es zu Impedanzänderungen während des Schweiß-/Prägevor-gangs kommen, denen dort durch eine automatische Impedanzanpassung und Frequenzänderung der benutzten Hochfrequenz entgegengewirkt wird.

Beim mobilen Einsatz in den Umgebungen, in denen solche Blutschlauchschweißgeräte typischerweise verwendet werden, ist eine unvermeidliche Abstrahlung von Hochfrequenzfeldern außerhalb ganz bestimmter, vorgegebener Frequenzen allerdings nicht zulässig und daher die Anpassung der Frequenz an die veränderte Impedanz keine Option. Der Erfindung liegt als technisches Problem die Bereitstellung eines Kunststoffschlauchschweißgerätes der eingangs genannten Art zugrunde, das eine zuverlässige, prozesssichere Verschweißung von Kunststoffschläuchen, wie Blutschläuchen, mit hoher Energieeffizienz ermöglicht und sich bei Bedarf als mobiles, handgehaltenes Gerät mit relativ niedrigem Gewicht und hohem Bedienkomfort ausführen lässt.

Die Erfindung löst dieses Problem durch die Bereitstellung eines Kunststoffschlauchschweißgeräts mit den Merkmalen des Anspruchs 1. Bei diesem Gerät enthält der HF-Energieversorgungsschaltkreis einen Hochfrequenzgenerator mit impedanzveränderlichem HF-Schwingkreis, wobei die Kapazität der zugehörigen Kondensatoreinheit und/oder die Induktivität der zugehörigen Spuleneinheit und/oder der ohmsche Widerstand des HF-Schwingkreises variabel einstellbar ist. Des Weiteren umfasst das erfindungsgemäße Gerät eine Impedanzsteuereinrichtung zur Konstanthaltung der Impedanz des HF-Energieversorgungsschaltkreises im Verlauf des jeweiligen Schweißvorgangs, wozu optional die Impedanz des HF-Energieversorgungsschaltkreises im Verlauf des jeweiligen Schweißvorgangs laufend gemessen wird. Bei dieser Option kann die Impedanzsteuereinrichtung zusätzlich als Impedanzmesseinrichtung ausgeführt sein.

Durch diese Auslegung ist das erfindungsgemäße Kunststoffschlauchschweißgerät in der Lage, die Impedanz des HF-Energieversorgungsschaltkreises, die sich durch die Zangenbewegung und das Verformen des Schlauchmaterials zwischen den Zangenbacken durch das Erhitzen während des Schweißvorgangs ändert, im Wesentlichen konstant zu halten, ohne dazu die Frequenz der für den Schweißvorgang benutzten Hochfrequenzstrahlung zu verändern. Dies ist unter anderem deshalb von großem Vorteil, weil in den Umgebungen, in denen solche Kunststoffschlauchschweißgeräte typischerweise verwendet werden, Hochfrequenzfelder mit anderen Frequenzen als ganz bestimmte, vorgegebene Frequenzen, wie der Frequenzwert der für den Schweißvorgang genutzten Hochfrequenz, im Allgemeinen unerwünscht oder sogar unzulässig sind.

In Weiterbildung der Erfindung beinhaltet die Kondensatoreinheit eine kapazitätsändernd bewegliche Kondensatorelektrode und/oder ein kapazitätsändernd bewegliches dielektrisches Element. Alternativ oder zusätzlich weist die Spuleneinheit ein induktivitätsverändernd bewegliches Element, z.B. ein Ferritelement, auf. Dies ermöglicht in konstruktiv einfacher Weise eine gewünschte Impedanzanpassung des HF-Energieversorgungsschaltkreises während des Schweißvorgangs, wie sie auch in einem mobilen, handgehaltenen Gerät realisierbar ist.

In einer weiteren Ausgestaltung ist die kapazitätsändernd bewegliche Kondensatorelektrode in der Schweißzange so angeordnet, dass sich die Kapazität ihrer Kondensatoreinheit bei einer schließenden Bewegung der HF-Elektroden gegenläufig zur Kapazität der HF-Elektroden ändert. Dadurch lässt sich in einfacher Weise eine Kapazitätskompensation und folglich Impedanzkonstanthaltung erzielen, indem diese beiden Kapazitäten elektrisch parallel geschaltet werden.

In Weiterbildung der Erfindung umfasst die Kondensatoreinheit eine Kapazitätsdiode, deren Kapazität sich elektronisch steuern lässt, vorliegend speziell dergestalt, dass ihre Kapazitätsänderung derjenigen der HF-Elektroden kompensierend entgegenwirkt.

In einer Ausgestaltung der Erfindung lässt sich die zur Konstanthaltung der Impedanz erforderliche Veränderung der Kondensatorkapazität oder Spuleninduktivität durch direkte mechanische Kopplung einer kapazitätsändernd beweglichen Kondensatorelektrode bzw. eines kapazitätsändernd beweglichen dielektrischen Elements oder eines induktivitätsändernd beweglichen Elements der Spuleneinheit realisieren.

In einigen Weiterbildungen der Erfindung weist das Kunststoffschlauchschweißgerät eine Kondensatoreinheit mit einer Kondensatorelektrode sowie einen ersten Kondensator auf. Der erste Kondensator enthält dabei eine äußere HF-Elektrode und eine mittlere HF-Elektrode, wobei vorzugsweise eine der HF-Elektroden als eine Gegenelektrode, der Kondensatorelektrode ausgebildet ist, also als zweite Elektrode eines mit der Kondensatorelektrode gebildeten Kondensators.

In einigen Weiterbildungen der Erfindung ist die mittlere HF-Elektrode zwischen der äußeren HF-Elektrode und der Kondensatorelektrode elektrisch mit demselben Potential wie die äußere HF-Elektrode verbunden, wobei eine Kapazität der HF-Elektroden und eine Kapazität der Kondensatoreinheit parallel verbunden sind. In einer alternativen Weiterbildung der Erfindung ist die mittlere HF-Elektrode zwischen der äußeren HF-Elektrode und der Kondensatorelektrode so angeordnet, dass eine Kapazität der HF-Elektroden und eine Kapazität der Kondensatoreinheit seriell verbunden sind.

Eine zusätzliche Kondensatorelektrode der Kondensatoreinheit kann durch die mittlere HF-Elektrode separat bereitgestellt sein, oder durch eine zusätzliche Elektrode, die vorzugsweise zwischen der mittleren HF-Elektrode und der Kondensatorelektrode angeordnet ist.

In einigen Weiterbildungen der Erfindung stellt die mittlere HF-Elektrode der Vorrichtung die kapazitätsändernd bewegliche Kondensatorelektrode dar. Die kapazitätsändernd bewegliche Kondensatorelektrode gemäß derartigen Weiterbildungen kann beweglich zwischen der äußeren HF-Elektrode und der Kondensatorelektrode angeordnet sein.

Vorteilhafterweise ist in einigen Weiterbildungen der vorliegenden Erfindung die mittlere HF-Elektrode mit einer Zangenbacke des Kunststoffschlauchschweißgerätes gekoppelt.

Wie ein Fachmann erkennen wird, gelten die oben dargelegten Weiterbildungen auch für Vorrichtungen, die nicht notwendigerweise Zangenbacken aufweisen, die jedoch andere Mittel aufweisen, die geeignet sind, die Elektroden für das Schlauchschweißgerät gemäß der Erfindung zu tragen. Zudem versteht es sich, dass die dargelegten Weiterbildungen grundsätzlich auch in Kombination miteinander ausführbar sind.

In Weiterbildung der Erfindung ist die Impedanzmesssteuereinrichtung zur Ermittlung eines Elektrodenabstands der Zangenbacken während des Schweißvorgangs ausgelegt. Dies kann beispielsweise durch Auswertung der laufend gemessenen Impedanz oder durch Verwenden eines lichtbasierten, induktiven oder resistiven Abstandssensors realisiert sein. Aus dem ermittelten Elektrodenabstand lassen sich weitere prozessrelevante Größen ableiten, wie die Abmessungen und das Material des zu verschweißenden Blutschlauchs und/oder die gewünschte endgültige Geometrie der Schweißstelle.

In Weiterbildung der Erfindung sind die HF-Elektroden der Zangenbacken thermisch isoliert ausgeführt. Dies trägt zur weiteren Optimierung der Energieeffizienz des Gerätes bei, indem Wärmeverluste an der Schweißstelle minimal gehalten werden.

In Weiterbildung der Erfindung beinhaltet das Gerät einen schnurlosen und/oder handgehaltenen Gerätekörper, der wenigstens den Zangenteil und den HF-Energieversorgungsschaltkreis enthält, vorzugsweise auch die Impedanzmesssteuereinrichtung. Dies trägt zu einem hohen Bedienkomfort des Gerätes bei. Die erfindungsgemäßen Maßnahmen und insbesondere die durch die spezielle Impedanzanpassung bewirkte hohe Energieeffizienz schaffen die Voraussetzung für die schnurlose Geräteausführung im Gegensatz zu den Blutschlauchschweißgeräten herkömmlicher Bauart, die eine schnur- bzw. kabelgebundene Ausführung erfordern.

In Weiterbildung der Erfindung umfasst das erfindungsgemäße Gerät als elektrische Energiequelle für den HF-Energieversorgungsschaltkreis eine wiederaufladbare Batterieeinheit, z.B. eine Lithium-Akkumulatoreinheit. Letztere lässt sich z.B. platz- und gewichtssparend in einem handgehaltenen Gerätekörper unterbringen und trägt ebenfalls zu einem hohen Bedien- und Einsatzkomfort bei. In weiterer Ausgestaltung beinhaltet das erfindungsgemäße Gerät eine Ladestation zum Ablegen des Gerätekörpers und zum Laden der Batterieeinheit. Dies ist insbesondere in Verbindung mit einer schnurlosen Ausführung des Gerätekörpers von Vorteil für den Bedien- und Einsatzkomfort des Gerätes.

Vorteilhafte Ausführungsformen der Erfindung sind in den Zeichnungen dargestellt und werden nachfolgend beschrieben. Hierbei zeigen:
- Fig. 1: eine Perspektivansicht eines Gerätekörpers eines Kunststoffschlauchschweißgerätes,
- Fig. 2: ein schematisches Blockdiagramm eines Kunststoffschlauchschweißgerätes mit einem HF-Generator mit variabler Schwingkreiskondensatorkapazität,
- Fig. 3: ein schematisches Blockdiagramm entsprechend Fig. 2 für eine Variante mit variabler Schwingkreisspuleninduktivität,
- Fig. 4: ein Elektrodenabstand-Zeit-Diagramm für einen typischen Schweißvorgang eines Kunststoffschlauchschweißgerätes,
- Fig. 5: eine Perspektivansicht einer Ladestation für zwei Gerätekörper nach Art von Fig. 1,
- Fig. 6: ein schematisches Blockschaltbild eines im Gerät von Fig. 1 verwendbaren HF-Schwingkreises in einer Ausführung mit einer zangenexternen Kondensatoreinheit variabler Kapazität zu Beginn eines Schweißvorgangs,
- Fig. 7: das Blockschaltbild von Fig. 6 am Ende eines Schweißvorgangs,
- Fig. 8: ein Blockschaltbild entsprechend Fig. 6 für eine Ausführungsvariante mit mechanischer Zangenankopplung einer Spuleneinheit variabler Induktivität zu Beginn eines Schweißvorgangs,
- Fig. 9: das Blockschaltbild von Fig. 8 am Ende eines Schweißvorgangs,
- Fig. 10: ein Blockschaltbild entsprechend Fig. 6 für eine zangenintegrierte Kondensatoreinheit variabler Kapazität zu Beginn eines Schweißvorgangs,
- Fig. 11: das Blockschaltbild von Fig. 10 am Ende eines Schweißvorgangs,
- Fig. 12: ein Blockschaltbild entsprechend Fig. 6 für eine Ausführungsvariante mit elektronisch steuerbarer Kapazitätsdiode zu Beginn eines Schweißvorgangs,
- Fig. 13: das Blockschaltbild von Fig. 12 am Ende eines Schweißvorgangs,
- Fig. 14: ein Blockschaltbild entsprechend Fig. 6 gemäß einer weiteren Ausführungsform der Erfindung zu Beginn eines Schweißvorgangs und
- Fig. 15: das Blockschaltbild von Fig. 14 am Ende eines Schweißvorgangs.

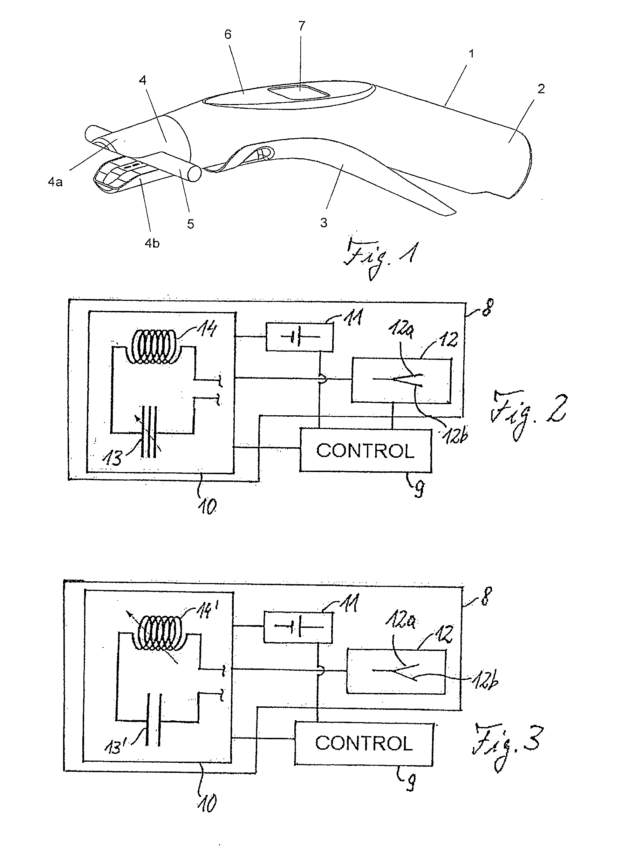

In einer vorteilhaften, exemplarischen Ausführungsart beinhaltet das erfindungsgemäße Kunststoffschlauchschweißgerät einen schnurlosen Gerätekörper. Fig. 1 zeigt einen derartigen schnurlosen Gerätekörper 1, der in dieser gezeigten Realisierung vom Benutzer mit einer Hand gehalten und bedient werden kann. Dazu weist der Gerätekörper 1 einen rückseitigen Griffteil 2 und einen als elektrischer Schalter fungierenden Bedienhebel 3 auf, der unterseitig mit den Fingern der den Gerätekörper haltenden Hand betätigt werden kann. Frontseitig weist der Gerätekörper 1 ein Zangenteil 4 auf, das zwei relativ zueinander bewegliche Zangenbacken 4a, 4b zum Einlegen und Zusammendrücken eines in Fig. 1 nur angedeuteten Kunststoffschlauchs 5 umfasst. Bei dem Kunststoffschlauch 5 kann es sich z.B. um einen Blutschlauch handeln, und das Gerät kann entsprechend als Blutschlauchschweißgerät ausgeführt sein. Dem unterseitigen Bedienhebel 3 liegt oberseitig ein Anzeigebereich 6 gegenüber, der zwischen dem rückwärtigen Griffbereich 2 und dem vorderseitigen Zangenteil 4 am Gerätekörper 1 ausgebildet ist. Der Anzeigebereich 6 umfasst ein Anzeigepanel 7, an dem gewünschte Informationen optisch angezeigt werden können.

In Fig. 2 sind schematisch ein elektrischer Hochfrequenz(HF)-Energieversorgungsschaltkreis 8 und eine Gerätesteuerung 9 gezeigt, wie sie z.B. für ein Blutschlauchschweißgerät mit dem schnurlosen, handgehaltenen Gerätekörper 1 von Fig. 1 verwendetwerden können. Der elektrische HF-Energieversorgungsschaltkreis 8 beinhaltet einen HF-Generator 10, eine elektrische Energiequelle 11 und eine Elektrodenanordnung 12. Der HF-Generator 10 ist von einer an sich herkömmlichen Bauart, wobei er einen impedanzveränderlichen HF-Schwingkreis mit einer Kondensatoreinheit 13 und einer Spuleneinheit 14 beinhaltet. Im gezeigten Bespiel ist die Kondensatoreinheit 13 als eine solche mit variabler, einstellbarer Kondensatorkapazität ausgeführt. Hierzu kann die Kondensatoreinheit 13 beispielsweise so ausgeführt sein, dass sie wenigstens zwei Kondensatorelektroden aufweist, von denen wenigstens eine gegenüber der anderen in Abstandsrichtung beweglich ist. Alternativ oder zusätzlich kann ein dielektrisches Element vorgesehen sein, das kapazitätsändernd im Zwischenraum zwischen zwei Kondensatorelektroden bewegt werden kann.

Die elektrische Energiequelle 11 ist vorzugsweise als wiederaufladbare Batterie- bzw. Akkumulatoreinheit realisiert, insbesondere kann hierfür eine Lithium-Akkumulatoreinheit bzw. ein Lithium-Batteriepack Verwendung finden, vorzugsweise ein solcher vom Lithiumionentyp, wie insbesondere ein Lithiumpolymer-Batteriepack oder ein LiFePO₄-Batteriepack. Vorteile einer solchen elektrischen Energiequelle sind ihr vergleichsweise niedriges Gewicht bei relativ hoher Speicherkapazität. In praktischen Ausführungsformen lassen sich damit Schweißkapazitäten von mehr als 500 Schweißvorgängen erzielen, bevor eine Wiederaufladung der Akkumulatoreinheit 11 erforderlich ist, bei einem Akkumulatorgewicht von höchstens ca. 200g, vorzugsweise höchstens 150g.

Die Elektrodenanordnung 12 umfasst zwei zugehörige, in Fig. 2 nur schematisch angedeutete HF-Elektroden 12a, 12b, von denen je eine in den beiden relativ zueinander beweglichen Zangenbacken des Blutschlauchschweißgerätes angeordnet ist, z.B. in den beiden Zangenbacken 4a, 4b des Zangenteils 4 des in Fig. 1 gezeigten Gerätekörpers 1. Auch der HF-Generator 10 und die elektrische Energiequelle 11 können im Gerätekörper 1 aufgenommen sein, d.h. der gesamte elektrische HF-Energieversorgungsschaltkreis 8 befindet sich dann im Gerätekörper 1. Die Zangenbacken 4a, 4b mit den HF-Elektroden 12a, 12b sind vorzugsweise auf minimale Energie-/Wärmeverluste ausgelegt. Dazu sind sie in nicht näher gezeigter, an sich herkömmlicher Weise mit einer thermischen Isolierung versehen, die dafür sorgt, dass von der Schweißstelle während des Schweißvorgangs abgehende Wärmeverluste minimal gehalten werden.

Der von der Energiequelle 11 gespeiste HF-Generator 10 liefert in an sich bekannter Weise für die Elektrodenanordnung 12 die zum Verschweißen eines zwischen die HF-Elektroden 12a, 12b eingelegten Blutschlauchs benötigte HF-Energie. Die Gerätesteuerung 9 steuert und überwacht den jeweiligen Schweißvorgang, wozu sie geeignet eingerichtet ist. Neben herkömmlichen Steuerungsmaßnahmen, auf die hier nicht näher eingegangen werden braucht, umfasst die Gerätesteuerung 9 erfindungsgemäß insbesondere eine Impedanzmess- und Impedanzsteuereinrichtung zur laufenden Messung und zur Konstanthaltung der Impedanz des HF-Energieversorgungsschaltkreises 8 im Verlauf des jeweiligen Schweißvorgangs. Die Gerätesteuerung 9 ist mit geeigneten Hardware-und Softwarekomponenten ausgerüstet, wie sich dies für den Fachmann bei Kenntnis der vorliegend erläuterten Funktionalitäten der Gerätesteuerung 9 versteht. Insbesondere beinhaltet die Gerätesteuerung 9 hierzu geeignete Rechnerkomponenten, z.B. einen herkömmlichen Mikrocontroller. In den Ausführungsformen mit dem schnurlosen und handgehaltenen Gerätekörper 1 von Fig. 1 kann die Gerätesteuerung 9 je nach Bedarf mit allen ihren Komponenten oder nur mit einem Teil ihrer Komponenten im Gerätekörper 1 angeordnet sein. Weiter alternativ kann die Gerätesteuerung 9 vollständig außerhalb des Gerätekörpers 1 angeordnet sein und mit dem im Gerätekörper 1 untergebrachten HF-Energieversorgungsschaltkreis 8 über eine geeignete, herkömmliche, drahtlose Schnittstelle in Kommunikationsverbindung stehen. Beispielsweise kann es sich um eine Bluetooth-Schnittstelle handeln.

Zur Durchführung eines Schweißvorgangs z.B. mit dem Gerät entsprechend den Fig. 1 und 2 wird der Blutschlauch 5 zwischen die Zangenbacken 4a, 4b und damit zwischen die HF-Elektroden 12a, 12b eingelegt, und dann werden die Zangenbacken 4a, 4b durch Betätigen des Bedienhebels 3 aufeinander zu bewegt, wodurch der Blutschlauch 5 zusammengedrückt wird. Gleichzeitig wird der HF-Energieversorgungsschaltkreis 8 aktiviert und versorgt den zu verschweißenden Blutschlauch 5 über die HF-Elektroden 12a, 12b an der Quetsch- bzw. Abklemmstelle zwischen den Zangenbacken 4a,4b mit der zum Aufschmelzen des Schlauchmaterials nötigen Hochfrequenzenergie. Durch das schließende Bewegen der Zangenbacken 4a, 4b und damit der HF-Elektroden 12a, 12b aufeinander zu ändert sich entsprechend der Elektrodenabstand der HF-Elektroden 12a, 12b, wodurch sich die Impedanz des HF-Energieversorgungsschaltkreises 8 ändern würde, wenn keine Gegenmaßnahmen ergriffen würden. Dazu kann auch die Verformung und Erwärmung des Schlauchmaterials an der Abklemmstelle zwischen den HF-Elektroden 12a, 12b beitragen. Eine solche Impedanzänderung hätte eine deutliche Verringerung der Energieeffizienz des Gerätes zur Folge. Zwar könnte dem durch anpassende Änderung der Frequenz der für den Schweißvorgang bereitgestellten Hochfrequenzstrahlung entgegengewirkt werden, dies kann jedoch zu unerwünschten Nebeneffekten führen.

Die Erfindung sieht daher eine andere Gegenmaßnahme vor, nämlich eine Konstanthaltung der Impedanz des HF-Energieversorgungsschaltkreises 8 über den Verlauf des Schweißvorgangs hinweg. Dazu erfasst die Impedanzmess- und -steuereinrichtung der Gerätesteuerung 9 über den Verlauf des jeweiligen Schweißvorgangs hinweg laufend den Momentanwert bzw. Istwert der Impedanz und sorgt für eine erforderliche Anpassung bzw. Nachführung durch Verstellen bzw. Nachführen der veränderlichen Kondensatorkapazität der Kondensatoreinheit 13. Die Gerätesteuerung 9 steuert dazu die Bewegung der Kondensatorelektrode bzw. des dielektrischen Elements so, dass die Impedanz des Energieversorgungsschaltkreises 8 zu jedem Zeitpunkt während des Schweißvorgangs konstant gehalten wird, was selbstverständlich die Möglichkeit beinhaltet, die Impedanz nur im Wesentlichen konstant zu halten und kleinere temporäre Abweichungen zuzulassen. Zur Impedanzmessung kann irgendeine der hierfür bekannten Messeinrichtungen zur komplexen Impedanzmessung verwendet werden.

Die Nachführung der Impedanz kann vorzugsweise durch mechanische Bewegung von Elementen erfolgen, welche die Impedanz induktiv, kapazitiv oder resistiv beeinflussen. Die Nachführung der Impedanz kann vorzugsweise durch elektronische Bauelemente ohne mechanische Bewegung erfolgen, wie z.B. Kapazitätsdioden. Je nach Bedarf und Anwendungsfall kann die Gerätesteuerung 9 aus der Messung der Impedanz und deren zeitlicher Änderung weitere interessierende Größen bzw. Informationen ableiten, wie den Elektrodenabstand der HF-Elektroden 12a, 12b, das Material des Blutschlauchs, die Dicke des Blutschlauchs vor und/oder während des Schweißvorgangs und/oder das Erkennen, ob ein Blutschlauch zwischen die HF-Elektroden 12a, 12b eingelegt ist. Häufig verwendete Blutschlauchmaterialien sind die Kunststoffe PVC und EVA, die bei gegebener HF-Energie unterschiedlich rasch aufschmelzen, so dass die Gerätesteuerung 9 aus der zeitlichen Änderung des Elektrodenabstands während des Schweißvorgangs und insbesondere schon in einer frühen Phase desselben feststellen kann, ob der eingelegte Blutschlauch aus PVC- oder EVA-Material ist. In der Implementierung mit dem Gerätekörper 1 von Fig. 1 kann die Gerätesteuerung 9 gewünschte Informationen an der Anzeigeeinheit 7 anzeigen, z.B. den Ladezustand der Akkumulatoreinheit 11 und/oder die mit dem momentanen Ladezustand voraussichtlich noch mögliche Anzahl an Schweißvorgängen.

Beispielsweise kann sich bei Verwendung einer Hochfrequenz von 40MHz eine durch die Impedanzänderung verursachte Verschiebung der Resonanzfrequenz auf ca. 36MHz ergeben, mit entsprechenden Folgen hinsichtlich Effizienzverlust. Durch die erfindungsgemäße Impedanzkonstanthaltung lässt sich die Hochfrequenz über den gesamten Verlauf des Schweißvorgangs hinweg zu jedem Zeitpunkt im Wesentlichen auf der Resonanzfrequenz von 40MHz halten. Entsprechend kann die Energieeffizienz optimal gehalten werden. Die durch die Gerätesteuerung 9 bewirkte Nachführung und damit Konstanthaltung der Impedanz des HF-Energieversorgungsschaltkreises 8 über den gesamten Verlauf des Schweißvorgangs hinweg ermöglicht so eine vergleichsweise gute und gleichbleibende Qualität der Schweißstelle mit minimalem Energieaufwand und optimierter Schweißdauer.

Dazu kann auch eine geeignete Endpunkterkennung des Schweißvorgangs beitragen. Es zeigt sich, dass eine optimale Qualität der Schweißstelle in der Regel dann vorliegt, wenn die Materialdicke an der Schweißstelle nicht zu dünn und nicht zu dick ist und in einem Bereich zwischen einer minimalen und einer maximalen Dicke liegt, der den Dickenwert der Schlauchwanddicke des Blutschlauchs beinhaltet. Mit anderen Worten wird für die fertiggestellte Schweißstelle ein Dickenwert angestrebt, der nicht zu weit unterhalb und nicht zu weit oberhalb der Dicke der Schlauchwand des Blutschlauchs liegt. Für einen typischen Blutschlauch mit einem Außendurchmesser von 4,2mm, einem Innendurchmesser von 2,8mm und folglich einer Schlauchwanddicke von 0,7mm erweist sich somit eine Schweißnahtdicke der fertiggestellten Verschweißungsstelle im Bereich von etwas unterhalb von 0,7mm bis etwas oberhalb von 0,7mm als optimal, z.B. im Bereich von ca. 0,5mm bis ca. 0,9mm.

Bei Bedarf kann der Gerätesteuerung 9 ein entsprechender Zielwert für die Materialdicke der fertigen Schweißstelle des Blutschlauchs in Form eines entsprechenden Sollwerts oder Sollbereichs vorgegeben werden, so dass die Gerätesteuerung 9 dann den Schweißvorgang beendet, sobald der ermittelte Istwert der Schweißnahtdicke im vorgegeben Sollbereich liegt bzw. den vorgegebenen Sollwert erreicht hat. Die Gerätesteuerung 9 kann den Istwert der Materialdicke des zusammengedrückten Blutschlauchs an der Verschweißungsstelle z.B. aus der laufend gemessenen Impedanz des HF-Energieversorgungsschaltkreises 8 oder aus einer laufenden direkten Messung des Elektrodenabstands bzw. des Abstands der Zangenbacken 4a, 4b voneinander ermitteln. Zur direkten Messung des Elektrodenabstands bzw. Zangenbackenabstands kann der Gerätesteuerung 9 ein entsprechender herkömmlicher Abstandssensor zugeordnet sein. Ein solcher Abstandssensor herkömmlichen Typs kann z.B. lichtbasiert oder vom induktiven oder resistiven Sensortyp sein.

Fig. 3 veranschaulicht eine Variante des Blutschlauchschweißgerätes gemäß Fig. 2, wobei für identische und funktionell äquivalente Komponenten gleiche Bezugszeichen verwendet sind und insoweit auf die obige Beschreibung zu Fig. 2 verwiesen werden kann. Bei der Gerätevariante von Fig. 3 erfolgt die Einstellung bzw. Nachführung der Impedanz des HF-Energieversorgungsschaltkreises 8 zwecks Konstanthaltung derselben durch anpassendes bzw. nachführendes Ändern/Verstellen der Induktivität einer insoweit modifizierten Spuleneinheit 14' des HF-Generators 10 anstelle der Spuleneinheit 14 mit unver-änderlicher Induktivität im Beispiel von Fig. 2 . In diesem Fall kann, wie gezeigt, eine insoweit modifizierte Kondensatoreinheit 13' mit konstant bleibender Kondensatorkapazität eingesetzt werden. Die variable Induktivität kann durch die Spuleneinheit 14' z.B. dadurch bereitgestellt werden, dass letztere ein induktivitätsverändernd bewegliches Element, vorzugsweise ein Ferritelement, aufweist, wie an sich bekannt, wobei die Gerätesteuerung 9 die Bewegung des Ferritelements so steuert, dass damit die Induktivität des HF-Energieversorgungsschaltkreises 8 konstant gehalten wird.

Im Übrigen gelten für das Gerät gemäß Fig. 3 die gleichen Eigenschaften und Vorteile, wie sie oben zum Gerät gemäß Fig. 2 erläutert wurden. In einer weiteren alternativen Gerätevariante können sowohl die Kondensatorkapazität als auch die Spuleninduktivität des HF-Generators 10 verändert werden, um für die gewünschte Konstanthaltung der Impedanz des HF-Energieversorgungsschaltkreises 8 zu sorgen. Dazu kann der HF-Generator 10 z.B. mit der Kondensatoreinheit 13 variabler Kondensatorkapazität von Fig. 2 und mit der Spuleneinheit 14' variabler Induktivität von Fig. 3 aufgebaut sein.

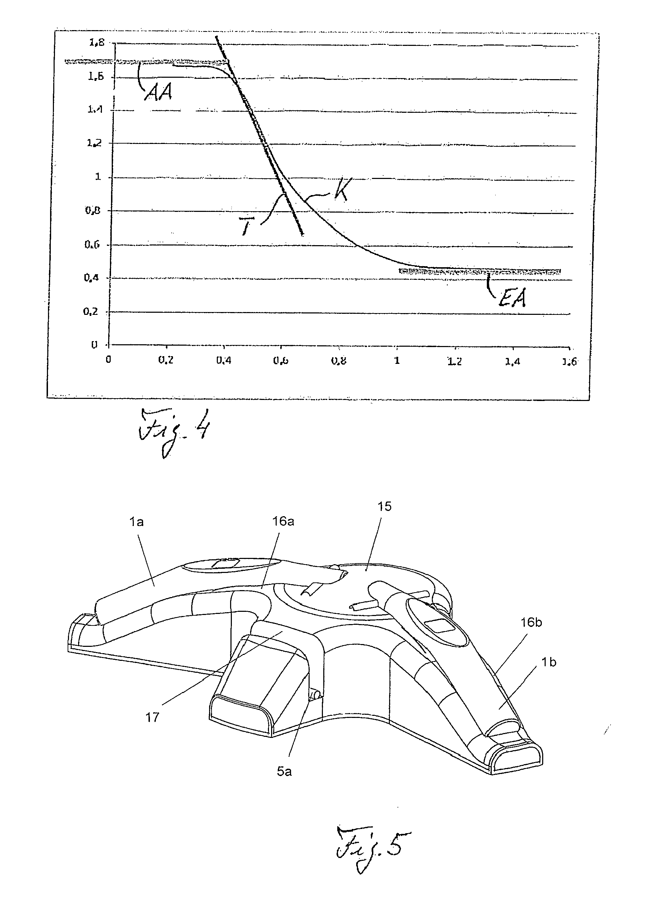

Fig. 4 veranschaulicht eine typische Kennlinie K für den Elektrodenabstand der HF-Elektroden 12a, 12b in Abhängigkeit von der Zeit für einen typischen Schweißvorgang. Die Gerätesteuerung 9 ist, wie erläutert, dafür eingerichtet, den Verlauf der Kennlinie K zu erfassen. Vor bzw. zu Beginn des Schweißvorgangs repräsentiert der erfasste Elektrodenabstand den Außendurchmesser und insoweit den Typ des eingelegten Blutschlauchs, ablesbar für die Gerätesteuerung 9 anhand einer zugehörigen horizontalen Anfangsasymptote AA des Kennlinienverlaufs K. Mit beginnendem Schweißvorgang beginnen sich die Zangenbacken des Zangenteils des Blutschlauchschweißgerätes und damit die HF-Elektroden einander anzunähern, wobei die zeitliche Änderung des Elektrodenabstands bei gegebener HF-Leistung durch die Erwärmungsrate bzw. Aufschmelzgeschwindigkeit des Blutschlauchmaterials bestimmt ist. Dementsprechend kann die Gerätesteuerung 9 aus der Steigung einer Tangente T an die Kennlinie K in einem ersten Schlaucherwärmungsabschnitts auf das Material des Blutschlauchs schließen, z.B. ob der Blutschlauch aus PVC oder EVA besteht.

Aus den somit vor bzw. während eines anfänglichen Abschnitts des Schweißvorgangs ermittelten Blutschlauchparametern kann die Gerätesteuerung 9 dann die gewünschte endgültige Schweißnahtdicke ermitteln, d.h. die optimale Materialdicke der herzustellenden Verschweißung. Die Gerätesteuerung 9 kann dies dafür nutzen, die HF-Heizleistung und den Endpunkt des Schweißvorgangs geeignet einzustellen bzw. vorzugeben. Entsprechend geht dann die Kennlinie K des zeitlichen Verlaufs des Elektrodenabstands gegen Ende des Schweißvorgangs in eine horizontale Endasymptote EA über, deren zugehöriger Elektrodenabstandswert die gewünschte Solldicke der Verschweißungsstelle des gegebenen Blutschlauchs repräsentiert.

Fig. 5 veranschaulicht eine Ladestation 15, die zwei Aufnahmen 16a, 16b zum Aufnehmen zweier Gerätekörper 1 a, 1 b entsprechend dem Gerätekörper 1 von Fig. 1 aufweist. Wenn der jeweilige Gerätekörper 1 a, 1 b in eine der Aufnahmen 16a, 16b der Ladestation 15 eingelegt ist, kann die in ihm befindliche Akkumulatoreinheit 11 durch die Ladestation 15 wieder elektrisch aufgeladen werden. Gleichzeitig dient die Ladestation 15 als Ablage für die Gerätekörper 1 a, 1 b. Die Ladestation 15 dient auf diese Weise als Docking-Station für die Gerätekörper 1a, 1b. Dabei lassen sich z.B. Ladezeiten von weniger als eine Stunde für eine Lithium-Akkumulatoreinheit realisieren. Im gezeigten Beispiel weist die Ladestation 15 zudem eine Schlitzaufnahme 17 auf, in der sich ein Blutschlauch 5a aufnehmen lässt.

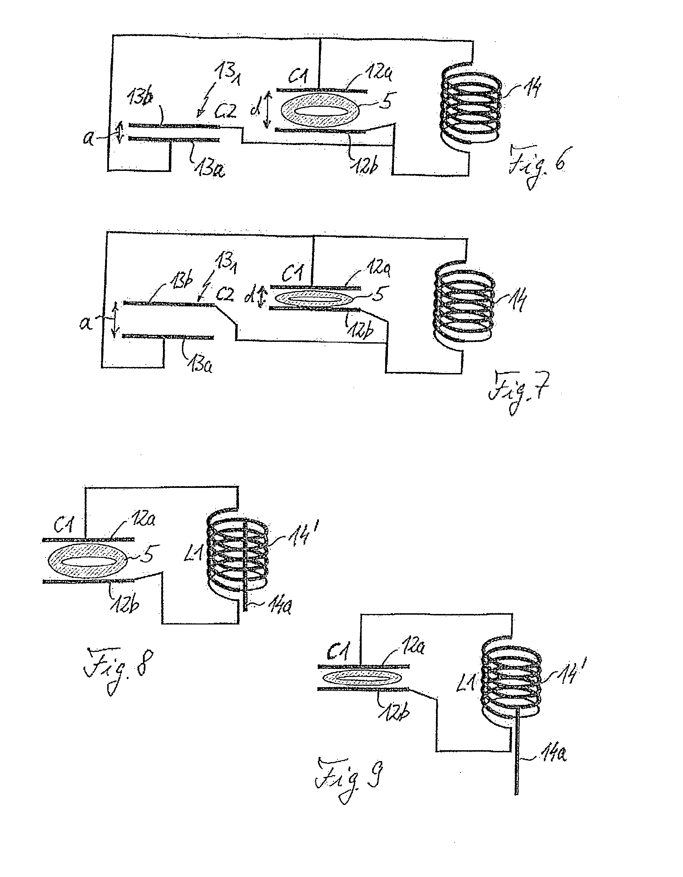

In den Fig. 6 bis 15 sind konkrete Ausführungsvarianten für die Konstanthaltung bzw. Nachführung der Impedanz des HF-Energieversorgungsschaltkreises für das erfindungsgemäße Kunststoffschlauchschweißgerät mit den hierzu interessierenden Komponenten schematisch dargestellt, jeweils im Zustand vor Beginn und am Ende eines Schweißvorgangs. Dies ist durch die HF-Elektroden 12a, 12b und den zwischen diese eingeklemmten Kunststoffschlauch 5 repräsentiert, wobei sich die in den Zangenbacken des Gerätes angeordneten HF-Elektroden 12a, 12b während des Schweißvorgangs aufeinander zu bewegen und dadurch ihren gegenseitigen Abstand d und in Folge davon ihre das Verhalten des HF-Schwingkreises beeinflussende elektrische Kapazität C1 vergrößern.

Im Ausführungsbeispiel der Fig. 6 und 7 ist in den HF-Schwingkreis außer der Spuleneinheit 14 eine Kondensatoreinheit 13₁ variabler Kondensatorkapazität C2 parallel zu der durch die HF-Elektroden 12a, 12b gebildeten Kapazität eingeschleift, d.h. je eine von zwei Elektroden 13a, 13b dieser Kondensatoreinheit 13₁ ist mit einer der beiden HF-Elektroden 12a, 12b elektrisch auf gleichem Potential liegend gekoppelt. Zusätzlich sind die beiden Kondensatorelektroden 13a, 13b, zwecks variabler Änderung ihres Abstands und damit der Kondensatorkapazität relativbeweglich angeordnet. Dies kann beispielsweise dadurch realisiert sein, dass eine der beiden Kondensatorelektroden 13a, 13b, z.B. die Elektrode 13b, an einem Gerätebauteil angeordnet ist, das sich bei der Zangenbewegung des Gerätes während des Schweißvorgangs bewegt, während die andere Kondensatorelektrode 13a an einem bei der Zangenbewegung nicht mitbewegten Gerätebauteil angeordnet ist. So kann die bewegliche Kondensatorelektrode 13b z.B. an einem Bedienhebel für die Zangenbewegung angeordnet sein und die andere Kondensatorelektrode 13a an einem gegenüberliegenden Gehäuseteil des Gerätes.

Wie in den Fig. 6 und 7 veranschaulicht, sind die Kondensatorelektroden 13a, 13b derart angeordnet, dass sie ihren gegenseitigen Abstand a vergrößern, wenn sich der Abstand d der HF-Elektroden 12a, 12b während des Schweißvorgangs verringert. Das Maß der Abstandsänderung der Kondensatorelektroden 13a, 13b ist dabei so gewählt, dass die durch die Abstandserhöhung abnehmende Kapazität C2 der Kondensatoreinheit 13₁ die von der Abstandsverringerung der HF-Elektroden 12a, 12b bewirkte Zunahme von deren Kondensatorkapazität C1 kompensiert, so dass die Gesamtkapazität C1+C2 des HF-Schwingkreises während des Schweißvorgangs im Wesentlichen konstant bleibt. In einer vorteilhaften Ausführungsform kann dies durch eine entsprechende mechanische Kopplung der beweglichen Kondensatorelektrode 13b an die Bewegung einer der Zangenbacken und damit deren HF-Elektrode 12b realisiert. Alternativ kann die Abstandsnachführung für die Kondensatorelektroden 13a, 13b abhängig von der erfassten Zangenbewegung bzw. der daraus resultierenden Impedanzänderung elektronisch erfolgen. Im Fall der mechanischen Kopplung kann bei Bedarf die in den Fig. 2 und 3 gezeigte Steuerung 9 entfallen bzw. entsprechend vereinfacht implementiert sein.

Die Fig. 8 und 9 veranschaulichen eine Ausführungsvariante, bei der die beim Schweißvorgang auftretende Änderung der Kapazität C1 der HF-Elektroden 12a, 12b durch eine Nachführung der Induktivität L1 der Spuleneinheit 14' mit variabler Induktivität kompensiert wird, wozu die Spuleneinheit 14' ein induktivitätsverändernd bewegliches Element in Form eines axial in der Spule beweglichen Ferritelements 14a aufweist. Das axiale Hineinbewegen des Ferritkerns 14a in die Spule und sein Herausbewegen aus derselben erfolgt wiederum abhängig von der Zangenbewegung während des Schweißvorgangs und damit der kapazitätsändernden Bewegung der HF-Elektroden 12a, 12b derart, dass die Gesamtimpedanz des HF-Schwingkreises im Wesentlichen konstant gehalten wird. Dazu wird der Ferritkern 14a beispielsweise, wie in den Fig. 8 und 9 illustriert, mit sich schließender Zangenbewegung weiter aus der Spule herausbewegt. Das Bewegen des Ferritkerns 14a abhängig von der Zangenschließbewegung kann wie oben zum Ausführungsbeispiel der Fig. 6 und 7 erläutert alternativ durch eine elektronische Steuerung oder durch eine mechanische Bewegungskopplung des Ferritkerns 14a an eine der Zangenbacken erfolgen, z.B. an die Zangenbacke, welche die HF-Elektrode 12b enthält.

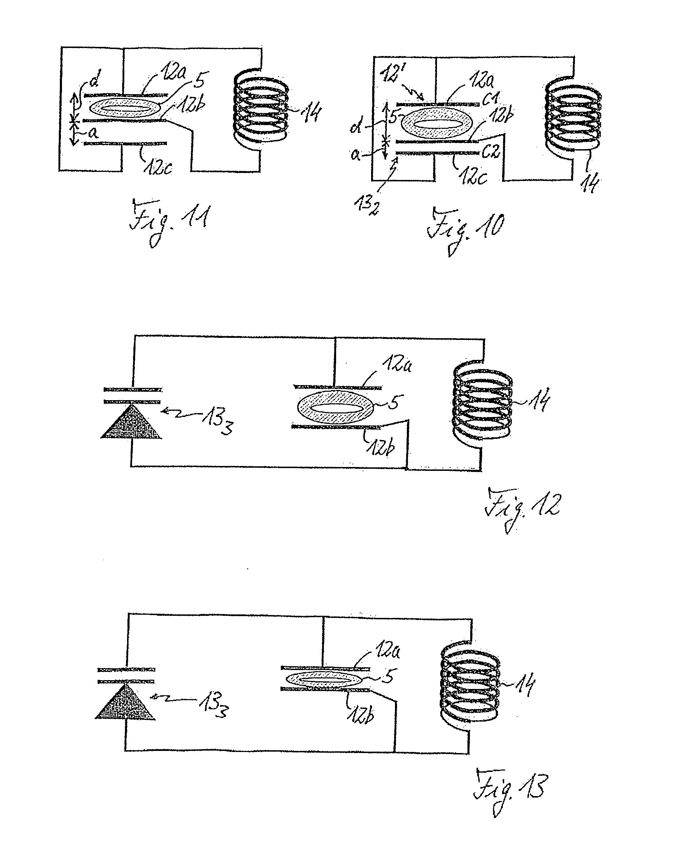

Die Fig. 10 und 11 veranschaulichen eine Modifikation des Ausführungsbeispiels der Fig. 6 und 7 dahingehend, dass eine Kondensatoreinheit 13₂ variabler Kapazität in die Zangenbacken des Gerätes integriert ist. Dazu ist das Zangenteil mit drei Zangenbacken 12a, 12b, 12c ausgeführt, von denen eine erste äußere Zangenbacke und eine mittlere Zangenbacke die beiden HF-Elektroden 12a, 12b tragen, während eine zweite äußere Zangenbacke die eine Kondensatorelektrode 12c dieser Kondensatoreinheit 13₂ trägt. Ihre andere Kondensatorelektrode wird von der HF-Elektrode 12b der mittleren Zangenbacke bereitgestellt. Die äußere Kondensatorelektrode 12c ist elektrisch mit der äußeren HF-Elektrode 12a auf dem gleichen Potential liegend gekoppelt. Dadurch liegt wie im Beispiel der Fig. 6 und 7 eine Parallelschaltung der Kapazität C1 der HF-Elektroden 12a, 12b mit der Kapazität C2 der Kondensatoreinheit 13₂ vor. Der zu verschweißende Schlauch 5 liegt zwischen den beiden die HF-Elektroden 12a, 12b tragenden Zangenbacken.

Somit bewegen sich bei einem Schweißvorgang die beiden HF-Elektroden 12a, 12b wieder aufeinander zu, wodurch sich ihre Kapazität C1 erhöht, gleichzeitig nimmt dabei aber der Abstand a zwischen den beiden Kondensatorelektroden 12b, 12c zu, so dass deren Kondensatorkapazität C2 abnimmt und dadurch die Gesamtkapazität C1+C2 wiederum im Wesentlichen konstant bleibt. Bei dieser Ausführungsvariante ändert somit die Bewegung der Zangenbacken selbst den Abstand a der variablen Kapazität 13₂ im Sinne einer Konstanthaltung der Impedanz des HF-Schwingkreises, so dass auch in diesem Beispiel eine entsprechende zusätzliche Steuerung nicht zwingend erforderlich ist.

Bei einer in den Fig. 12 und 13 veranschaulichten Ausführungsvariante dient eine elektronisch steuerbare Kapazitätsdiode 13₃ als Kondensatoreinheit mit variabler Kapazität. Das Schema in Fig. 13 zeigt ein prinzipielles Schema ohne Berücksichtigung der maximalen Spannungen. In einer anderen Ausführung kann der durchstimmbare Schwingkreis induktiv oder kapazitiv vom Lastschwingkreis getrennt sein. Deren Kapazität kann durch eine der Schwingspannung des HF-Schwingkreises überlagerte, variable Gleichspannung verändert werden, wie dem Fachmann an sich bekannt, was daher hier keiner näheren Erläuterungen bedarf. Eine nicht gezeigte Steuerung bzw. Regelung erfasst die Ist-Impedanz des HF-Schwingkreises bzw. einen dafür verantwortlichen Parameter, wie den Abstand d der HF-Elektroden 12a, 12b, und steuert die Kapazitätsdiode 13₃ mit der zur Konstanthaltung der Impedanz des HF-Schwingkreises durch entsprechende Kapazitätsänderung notwendigen Gleichspannung an. Auch hier ist die Kapazität der Kapazitätsdiode 13₃ wiederum elektrisch zur Kapazität der HF-Elektroden 12a, 12b parallel in den HF-Schwingkreis eingeschleift. Im Übrigen gelten für diese Ausführungsvariante die oben zu den Ausführungsbeispielen der Fig. 6 und 7 bzw. 10 und 11 gemachten Erläute-rungen in gleicher Weise.

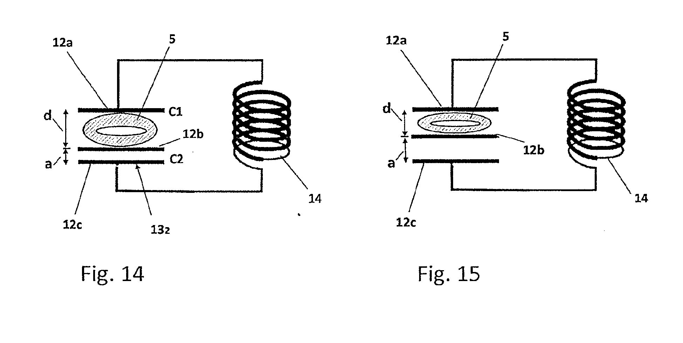

Die Figuren 14 und 15 veranschaulichen eine weitere Modifikation des Ausführungsbeispiels der Fig. 6 und 7 dahingehend, dass eine Kondensatoreinheit 13₂ variabler Kapazität in die Zangenbacken des Gerätes integriert ist. Diese Modifikation ist generell ähnlich der in Fig. 10 und 11 gezeigten. Gleiche Bezugszeichen bezeichnen dabei gleiche oder ähnliche Komponenten wie in den Figuren 10 oder 11, deren Beschreibung hier nicht wiederholt wird. Die in Fig. 14 und 15 gezeigte Modifikation der erfindungsgemäßen Vorrichtung unterscheidet sich dadurch von der Ausführungsform, die in Fig. 10 und 11 gezeigt ist, dass die von den RF Elektroden 12a, 12b gebildete Kapazität C1 mit der von den RF Elektroden 12b, 12c gebildeten Kapazität C2 in Serie geschaltet ist. Funktion und Ablauf des Schweißvorgangs sind identisch zu dem Vorgang, der bezüglich den Fig. 10 und 11 beschrieben ist. In diesem Beispiel bewegt sich nur die mittlere Elektrode 12b, wobei die Gesamtkapazität der Schweißzange konstant bleibt. In dieser Modifikation zeigt Fig.14 den Zustand vor Beginn des Schweißvorgangs, Fig. 15 nach Beendigung des Schweißvorgangs.

In nicht gezeigten Ausführungen der Erfindung ist das Blutschlauchschweißgerät als stationäres Standgerät ausgeführt. In weiteren alternativen Ausführungsformen der Erfindung weist das Blutschlauchschweißgerät einen handgehaltenen Gerätekörper auf, der weitestgehend demjenigen von Fig. 1 entspricht, jedoch kabelgebunden ausgeführt ist. In diesem Fall sind die im Gerätekörper aufgenommenen Gerätekomponenten über eine entsprechende Kabelverbindung mit den übrigen, außerhalb des Gerätekörpers angeordneten Komponenten des Blutschlauchschweißgerätes verbunden. Je nach Anwendungsfall kann z.B. die gesamte oder ein Teil der Gerätesteuerung und/oder die elektrische Energiequelle außerhalb des Gerätekörpers angeordnet sein.

Wie die oben erwähnten Ausführungsbeispiele verdeutlichen, stellt die Erfindung ein vorteilhaftes Blutschlauchschweißgerät zur Verfügung, das sich bei Bedarf als mobiles Gerät mit geringem Gewicht und einem schnurlosen Gerätekörper ausführen lässt, wobei das erfindungsgemäße Blutschlauchschweißgerät eine hohe Energieeffizienz und Prozessgenauigkeit für den Schweißvorgang ermöglicht. Dazu trägt insbesondere eine laufende Impedanzkonstanthaltung der für den Schweißvorgang bereitgestellten HF-Energie über den gesamten Verlauf des Schweißvorgangs hinweg bei. Für das erfindungsgemäße Gerät kann eine Akkumulatoreinheit mit geringem Gewicht benutzt werden. Frequenzänderungen der Hochfrequenzstrahlung während des Schweißvorgangs können vermieden werden.

## Patentansprüche

1. Kunststoffschlauchweißgerät, insbesondere Blutschlauchschweißgerät, mit
- einem Zangenteil (4), das zwei relativ zueinander bewegliche Zangenbacken (4a, 4b) zum Einlegen und Zusammendrücken eines Blutschlauchs beinhaltet, wobei die Zangenbacken Hochfrequenz(HF)-Elektroden (12a, 12b) beinhalten,
- einem elektrischen HF-Energieversorgungsschaltkreis (8) mit einem HF-Generator (10), der einen impedanzveränderlichen HF-Schwingkreis mit einer Kondensatoreinheit (13, 13') und einer Spuleneinheit (14, 14') beinhaltet, und mit den HF-Elektroden, wobei die Kapazität der Kondensatoreinheit und/oder die Induktivität der Spuleneinheit und/oder der ohmsche Widerstand des HF-Schwingkreises variabel einstellbar ist, und
- einer Impedanzsteuereinrichtung (9) zur laufenden Messung und zur Konstanthaltung der Impedanz des HF-Energieversorgungsschaltkreises im Verlauf des jeweiligen Schweißvorgangs.

2. Kunststoffschlauchschweißgerät nach Anspruch 1, weiter **dadurch gekennzeichnet, dass** die Kondensatoreinheit eine kapazitätsändernd bewegliche Kondensatorelektrode und/oder ein kapazitätsändernd bewegliches dielektrisches Element aufweist und/oder die Spuleneinheit ein induktivitätsändernd bewegliches Element, insbesondere ein Ferritelement, aufweist.

3. Kunststoffschlauchschweißgerät nach Anspruch 1 oder 2, weiter **dadurch gekennzeichnet, dass** die kapazitätsändernd beweglichen Kondensatorelektroden in der Schweißzange so angeordnet sind, dass eine schließende Bewegung der Schweißelektroden durch eine gegenläufige Bewegung der seriell oder parallel geschalteten Kapazität der Kondensatoreinheit ausgeglichen wird.

4. Kunststoffschlauchschweißgerät nach einem der Ansprüche 1 bis 3, weiter **dadurch gekennzeichnet, dass** der Schwingkreis eine elektrisch steuerbare Kapazitätsdiode (13₃) enthält.

5. Kunststoffschlauchschweißgerät nach Anspruch 2 oder 3, weiter **dadurch gekennzeichnet, dass** die kapazitätsändernd bewegliche Kondensatorelektrode mechanisch mit einer der die HF-Elektroden enthaltenden Zangenbacken gekoppelt ist.

6. Kunststoffschlauchschweißgerät nach einem der Ansprüche 3 bis 5, weiter **dadurch gekennzeichnet, dass** das Kunststoffschlauchschweißgerät eine Kondensatoreinheit (13₂) mit einer Kondensatorelektrode (12c) sowie einen ersten Kondensator (C1) aufweist, der eine äußere HF-Elektrode (12a) und eine mittlere HF-Elektrode (12b) aufweist, wobei vorzugsweise eine der HF-Elektroden als eine Gegenelektrode der Kondensatorelektrode (12c) ausgebildet ist.

7. Kunststoffschlauchschweißgerät nach Anspruch 6, weiter **dadurch gekennzeichnet, dass** die mittlere HF-Elektrode (12b) zwischen der äußeren HF-Elektrode (12a) und der Kondensatorelektrode (12c) angeordnet ist, wobei die Kondensatorelektrode (12c) elektrisch mit einem selben Potential gekoppelt ist wie die äußere HF-Elektrode (12a), derart, dass eine Kapazität (C1) der HF-Elektroden (12a, 12b) und eine Kapazität der Kondensatoreinheit (13₂) parallel verbunden sind.

8. Kunststoffschlauchschweißgerät nach Anspruch 6, weiter **dadurch gekennzeichnet, dass** die mittlere HF-Elektrode (12b) zwischen der äußeren HF-Elektrode (12a) und der Kondensatorelektrode (12c) derart angeordnet ist, dass eine Kapazität (C1) der HF-Elektroden (12a, 12b) und eine Kapazität der Kondensatoreinheit (13₂) in Serie verbunden sind.

9. Kunststoffschlauchschweißgerät nach Anspruch 6 oder 7, weiter **dadurch gekennzeichnet, dass** die mittlere HF-Elektrode (12b) die kapazitätsändernd bewegliche Kondensatorelektrode ist, die beweglich zwischen der äußeren HF-Elektrode (12a) und der Kondensatorelektrode (12c) angeordnet ist.

10. Kunststoffschlauchschweißgerät nach einem der Ansprüche 6 bis 9, weiter **dadurch gekennzeichnet, dass** die mittlere HF-Elektrode (12b) mit einer Zangenbacke gekoppelt ist..

11. Kunststoffschlauchschweißgerät nach einem der vorhergehenden Ansprüche, weiter **dadurch gekennzeichnet, dass** die Impedanzsteuereinrichtung zur Ermittlung eines Elektrodenabstands der Zangenbacken vor und/oder während des jeweiligen Schweißvorgangs ausgelegt ist.

12. Kunststoffschlauchschweißgerät nach einem der vorhergehenden Ansprüche, weiter **dadurch gekennzeichnet, dass** die HF-Elektroden der Zangenbacken thermisch isoliert ausgeführt sind.

13. Kunststoffschlauchschweißgerät nach einem der vorhergehenden Ansprüche, weiter **dadurch gekennzeichnet, dass** es einen schnurlosen und/oder handgehaltenen Gerätekörper (1) aufweist, der wenigstens das Zangenteil und den elektrischen HF-Energieversorgungsschaltkreis enthält.

14. Kunststoffschlauchschweißgerät nach einem der vorhergehenden Ansprüche, weiter **gekennzeichnet durch** eine wiederaufladbare Batterieeinheit (11) als elektrische Energiequelle für den HF-Energieversorgungsschaltkreis.

15. Kunststoffschlauchschweißgerät nach Anspruch 14, weiter **gekennzeichnet durch** eine Ladestation zum Ablegen des Gerätekörpers und zum Laden der Batterieeinheit.
